(19) Europäisches Patentamt
European Patent Office
Office européen des brevets

(11) **EP 2 307 072 B1**

(12) **EUROPÄISCHE PATENTSCHRIFT**

(45) Veröffentlichungstag und Bekanntmachung des Hinweises auf die Patenterteilung:
**20.06.2012   Patentblatt 2012/25**

(21) Anmeldenummer: **09772147.6**

(22) Anmeldetag: **30.06.2009**

(51) Int Cl.:
**A61M 1/28** (2006.01)

(86) Internationale Anmeldenummer:
**PCT/EP2009/004722**

(87) Internationale Veröffentlichungsnummer:
**WO 2010/000447 (07.01.2010 Gazette 2010/01)**

(54) **VORRICHTUNG ZUR PERITONEALDIALYSE**

DEVICE FOR PERITONEAL DIALYSIS

SYSTÈME POUR DIALYSES PÉRITONÉALES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **04.07.2008   DE 102008031637
31.07.2008   DE 102008035742**

(43) Veröffentlichungstag der Anmeldung:
**13.04.2011   Patentblatt 2011/15**

(73) Patentinhaber: **Fresenius Medical Care Deutschland GmbH
61352 Bad Homburg (DE)**

(72) Erfinder:
• **HEDMANN, Frank
97332 Volkach (DE)**
• **KLATTE, Stephan
31582 Nienburg (Weser) (DE)**

(74) Vertreter: **Laufhütte, Dieter et al
Lorenz-Seidler-Gossel
Widenmayerstrasse 23
80538 München (DE)**

(56) Entgegenhaltungen:
WO-A-01/30422      WO-A-95/27520
US-A- 4 368 737      US-A- 4 898 576
US-A- 5 006 997      US-A- 5 141 493

**Beschreibung**

Die Erfindung betrifft eine Vorrichtung zur Peritonealdialyse mit einer Einrichtung zur regelmäßigen Abgabe und Wiederaufnahme von Dialysat, die eine Leitung mit einem Katheter umfaßt.

Die Peritonealdialyse ist eine Variante der künstlichen Blutwäsche. Hier wird das gut durchblutete Bauchfell (Peritonium) des Patienten als körpereigene Filtermembran verwendet. Über einen Katheter wird Dialysat in die Bauchhöhle eingeführt. Nach dem Prinzip der Osmose werden Harnbestandteile dem Blut entzogen und gelangen in die Bauchhöhle. Nach einigen Stunden wird das Dialysat mit den Harnbestandteilen wieder aus der Bauchhöhle ausgelassen.

Zur Durchführung der Peritonealdialyse gibt es grundsätzlich verschiedene Möglichkeiten. Bei der kontinuierlichen ambulanten Peritonealdialyse (CAPD) wechseln die Patienten selbst etwa vier bis fünfmal am Tag das Dialysat. Bei der automatischen Peritonealdialyse (APD) übernimmt eine Vorrichtung, der sogenannte Cycler, den automatischen Beutelwechsel über Nacht, so dass der Patient tagsüber noch unabhängig ist.

Bei der automatischen Peritonealdialyse, bei der die Bauchhöhle mit Hilfe des vorgenannten Cyclers befüllt wird, wird üblicherweise über eine Volumensteuerung darauf geachtet, dass dem Patienten nur ein maximales Füllvolumen verabreicht wird. Dieses maximale Füllvolumen beträgt bei einem durchschnittlichen Erwachsenen beispielsweise 3.500 ml. Üblicherweise werden standardisierte Werte für das Füllvolumen verwendet, da der Aufwand für eine experimentelle Bestimmung des patientenindividuellen Füllvolumens vermieden werden soll.

Bei der Peritonealdialyse benötigt der Patient einen Zugang zur Bauchhöhle des Patienten, den sogenannten Patientenkatheter, über den der Flüssigkeitsaustausch erfolgt. Dieser Katheter mündet mit einem Ende in die Bauchhöhle des Patienten, auf der anderen Seite - außerhalb des Patienten - befindet sich ein Patientenkonnektor, der eine Ankoppelungsmöglichkeit an ein Schlauchsystem bietet. Ein solcher Gegenstand ist in US-5 141 493 beschrieben. Mit der Zeit kann sich der Katheter durch Ablagerungen zusetzen, so dass seine Funktion eingeschränkt sein kann.

Um die uneingeschränkte Funktion zu gewährleisten wird derzeit der Katheterwiderstand vom behandelnden Arzt manuell durchgeführt. Dabei wird eine Kenngröße für den Katheterwiderstand aus der für den Auslauf eines bestimmten Volumenstroms Flüssigkeit benötigten Zeit.

Aufgabe der vorliegenden Erfindung ist es nun, eine Vorrichtung zur Peritonealdialyse derart weiterzubilden, dass von der Vorrichtung automatisch der Katheterwiderstand bestimmbar ist.

Erfindungsgemäß wird diese Aufgabe durch die Kombination der Merkmale des Anspruchs 1 gelöst. Demnach ist eine Vorrichtung zur Peritonealdialyse mit einer Einrichtung zur regelmäßigen Abgabe und Wiederaufnahme von Dialysat (dem sogenannten Cycler) vorgesehen, die eine Leitung mit einem Katheter umfaßt. Erfindungsgemäß ist hier am Cycler zusätzlich ein Meßbeutel vorhanden, in welchem Dialysat aufnehmbar ist. Dabei sind erste Mittel vorgesehen sind, mit denen der statische Druck in der Leitung zwischen Katheter und Meßbeutel bestimmbar ist. Weiterhin sind zweite Mittel vorgesehen, mittels derer der Dialysatvolumenstrom für eine bestimmte Zeit in den Meßbeutel leitbar ist. Schließlich sind dritte Mittel vorhanden, mit denen das Volumen des in den Meßbeutel geleiteten Dialysats bestimmbar ist und schließlich sind vierte Mittel vorhanden, über die der Katheterwiderstand im wesentlichen aus den mittels der ersten bis dritten Mitteln bestimmten Werten errechenbar ist.

Bevorzugte Ausgestaltungen der Erfindung ergeben sich aus den sich an den Hauptanspruch anschließenden Unteransprüchen.

Demnach können die ersten Mittel einen Drucksensor umfassen, mittels dem der statische Druck ermittelbar ist.

Die zweiten Mittel weisen vorteilhaft mindestens ein zeitsteuerbares Ventil auf, um durch automatische Ventilstellung für einen vorgegebenen Meßzeitraum das Dialysat in den Beutel zu leiten.

Des weiteren weisen die dritten Mittel vorzugsweise eine Pumpe und eine Bilanziereinheit auf, um das im Beutel aufgefangene Dialysatvolumen dadurch zu bestimmen, dass es aus dem Meßbeutel wieder herausgefördert und durch die Bilanziereinheit geleitet wird.

Vorteilhaft ist in der Leitung zwischen Katheter und Meßbeutel ein erstes Ventil, ein Drucksensor, eine Pumpe und ein zweites Ventil hintereinander angeordnet.

Besonders vorteilhaft zweigt von der Leitung vor dem ersten Ventil eine Bypassleitung ab, in der ein drittes Ventil angeordnet ist. Diese Bypassleitung mündet nach dem zweiten Ventil wieder in die Leitung.

Mit der erfindungsgemäßen Vorrichtung kann die Messung des Katheterwiderstandes automatisch durchgeführt werden. Dabei ist es denkbar, den Katheterwiderstand zu Beginn der Behandlung zu überprüfen um auf eventuelle Abweichungen von einem vorher bestimmten erwarteten Wert reagieren zu können. Auch eine Speicherung der Kenngröße für den Katheterwiderstand über mehrere Messungen kann Aufschluß über die Veränderung der Kathetereigenschaft geben. Hier kann es sich beispielsweise um eine Meßreihe aus dem jeweils ermittelten Wert vor einer Behandlung handeln. Derartige Meßreihen können auf einer Patientenkarte abgespeichert werden und vor jeder Behandlung mit dem aktuellen gemessen Wert verglichen werden.

Mit der erfindungsgemäßen Vorrichtung lässt sich der Katheterwiderstand gravimetrisch wie folgt ermitteln:

1. Schritt: Höheneinmessung des Patienten, das heißt Bestimmung des statischen Patientendrucks.

2. Schritt: Gravimetrische Entleerung des Dialysats aus dem Patienten in einen Meßbeutel, wobei der Patient oberhalb des Beutels liegen muss.

3. Schritt: Höheneinmessung des Beutels dadurch, dass nach einer bestimmten Zeit der Dialysatvolumenstrom gestoppt wird und der Beutel ebenfalls statisch eingemessen wird.

4. Schritt: Volumenbilanzierung des Inhalts des Meßbeutels bei Entleerung des Meßbeutels.

5. Schritt: Aus den ermittelten Parametern Volumen und Zeit lässt sich der Katheterflußwiderstand errechnen.

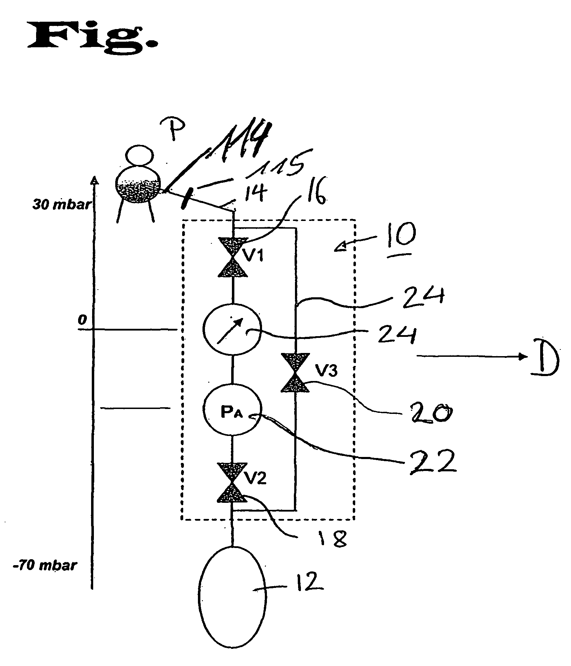

Weitere Einzelheiten und Vorteile der Erfindung werden anhand eines in der Zeichnung dargestellten Ausführungsbeispiels näher erläutert.

Die einzige Figur zeigt schematisch eine erfindungsgemäße Vorrichtung, wobei hier die für die Beschreibung der Vorrichtung funktionsnotwendigen Bestandteile als Symbole dargestellt sind.

Ein Patient P ist im hier dargestellten Ausführungsbeispiel mit einem ausreichenden Volumen an Dialysat befüllt, das beispielsweise 2000 ml umfassen kann.

Der Patient P ist verbunden mit dem sogenannten Cycler 10, das heißt der Vorrichtung zum regelmäßigen Abgeben und wieder aufnehmen von Dialysat. Am Cylcer 10 ist ein Meßbeutel 12 mit bekanntem Volumen angehängt. Der Meßbeutel ist unterhalb (tiefer) als der Patient angeordnet. Das Volumen des Meßbeutels beträgt im hier dargestellten Ausführungsbeispiel zu Beginn der Messung 0 ml. Der Meßbeutel 12 ist über eine Leitung 14 mit dem Patientenkatheter 114 verbunden. Der Flußwiderstand des Leitungssystems ist bekannt. Im hier dargestellten Ausführungsbeispiel kann dieser Flußwiderstand - ohne den Flußwiderstand des Patientenkatheters 114 - $R_{System}$ = 0,2 mbar x min/ml betragen.

Während der im folgenden Ausführungsbeispiel beschriebenen Messung soll die Lage des Patienten P zum Meßbeutel 12 möglichst konstant sein.

Zu Beginn wird der statische Druck des Patienten P ermittelt. Hierzu wird das erste Ventil 16 geöffnet, während das zweite Ventile 18 sowie das dritte Ventil 20 geschlossen bleiben. Im hier dargestellten Ausführungsbeispiel beträgt der statische Druck $P_{pat\ stat}$ = 30 mbar.

Für die Widerstandsbestimmung wird das Dialysat vom Patienten für eine bestimmte Zeit x gravimetrisch, das heißt durch Schwerkraft, in dem Meßbeutel 12 entleert. Dies kann entweder über das erste Ventil 16, die Pumpenkammer der in der Leitung ebenfalls angeordneten Pumpe 22 sowie das zweite Ventil 18 einerseits oder über eine Bypassleitung 24 und das in dieser angeordnete dritte Ventil 20 erfolgen. Dabei wird die Zeitspanne x so gewählt, dass eine Komplettentleerung des Patienten vermieden wird. Im hier dargestellten Ausführungsbeispiel wird von einem maximalen Volumenstrom von 300 ml/min ausgegangen. Um ein gutes Meßergebnis zu erzielen wird daher eine Meßzeit von x = 3 min gewählt. Dies führt zu einem erwarteten Gesamtvolumen im Meßbeutel von ca. 900 ml, so dass eine Komplettentleerung des Patienten mit Dialysat ausgeschlossen werden kann.

Im nächsten Schritt wird der statische Druck des Beutels ermittelt. Hierzu wird das zweite Ventil 18 geöffnet, während das erste Ventil 16 und das dritte Ventil 20 geschlossen sind. Der statische Druck des Beutels wird - wie zuvor auch schon der statische Druck des Patienten - über einen Drucksensor 24, der in der Leitung 214 angeordnet ist, gemessen. Im hier dargestellten Ausführungsbeispiel beträgt der gemessene statische Druck $P_{beutel\ stat}$ = 70 mbar.

Um das geflossene Volumen an Dialysat zu ermitteln, wird der Meßbeutel mit Hilfe der Pumpe 22 in eine hier nicht dargestellte Drainage D (vgl. Pfeil) über einen ebenfalls nicht näher dargestellten Flußweg entleert und über eine hier nicht näher dargestellte Bilanzierungseinheit bilanziert bzw. gemessen. Im hier vorliegenden Ausführungsbeispiel beträgt das tatsächlich gemessene Volumen aus dem Meßbeutel $V_{beutel}$ = 600 ml.

Über eine hier ebenfalls nicht näher dargestellte Recheneinheit wird aus den gemessenen bzw. festgelegten Werten der Widerstand des Patientenkatheters 114 wie folgt errechnet:

1. Schritt: Berechnung der Druckdifferenz zwischen Patient und Beutel:

$$P_{stat\ ges} = P_{pat\ stat} + \left| P_{beutel\ stat} \right| = 30\,mbar + \left| -70\,mbar \right| = 100\,mbar$$

2. Schritt: Berechnung der Flußrate:

$$Q = V_{beutel} / T = 600ml / 3\min = 200ml / \min$$

3. Schritt: Berechnung des Gesamtleitungswiderstandes des Systems incl. Widerstand des Patientenkatheters:

$$R = P_{stat\ ges} / Q = 100mbar / 200ml / \min = 0,5mbar * \min/ ml$$

4. Schritt: Berechnung des Patientenkatheterwiderstandes:

$$R_{Katheter} = R - R_{System} = 0,5mbar * \min/ ml - 0,2mbar * \min/ ml$$

$$R_{Katheter} = 0,3mbar * \min/ ml$$

**Patentansprüche**

1. Vorrichtung zur Peritonealdialyse mit einer Einrichtung zur regelmäßigen Abgabe und Wiederaufnahme von Dialysat, die eine Leitung mit einem Katheter umfaßt, wobei ein Meßbeutel vorhanden ist, in dem Dialysat aufnehmbar ist, dass erste Mittel vorgesehen sind, mit denen der statische Druck in der Leitung zwischen Katheter und Meßbeutel bestimmbar ist, dass zweite Mittel vorgesehen sind, mittels derer der Dialysatvolumenstrom für eine bestimmte Zeit in den Meßbeutel leitbar ist, dass dritte Mittel vorhanden sind, mit denen das Volumen des in den Meßbeutel geleiteten Dialysats bestimmbar ist; **dadurch gekennzeichnet, dass** vierte Mittel vorhanden sind, über die der Katheterwiderstand aus den mittels der ersten bis dritten Mitteln bestimmten Werten errechenbar ist.

2. Vorrichtung nach Anspruch 1, **dadurch gekennzeichnet, dass** die ersten Mittel einen Drucksensor umfassen.

3. Vorrichtung nach Anspruch 1 oder 2, **dadurch gekennzeichnet, dass** die zweiten Mittel mindestens ein zeitsteuerbares Ventil aufweisen.

4. Vorrichtung nach einem der Ansprüche 1 bis 3, **dadurch gekennzeichnet, dass** die dritten Mittel eine Pumpe und eine Bilanziereinheit zur Bestimmung des Dialysatvolumens aufweisen.

5. Vorrichtung nach einem der Ansprüche 1 bis 4, **dadurch gekennzeichnet, dass** in der Leitung zwischen Katheter und Meßbeutel ein erstes Ventil, ein Drucksensor, eine Pumpe und ein zweites Ventil angeordnet sind.

6. Vorrichtung nach Anspruch 5, **dadurch gekennzeichnet, dass** von der Leitung vor dem ersten Ventil eine Bypassleitung abzweigt, in der ein drittes Ventil angeordnet ist, und dass die Bypassleitung nach dem zweiten Ventil wieder in die Leitung mündet.

7. Vorrichtung nach einem der vorhergehenden Ansprüche, wobei mittels der Vorrichtung der Katheterwiderstand mit folgenden Schritten automatisch bestimmbar ist:

   • Höheneinmessung des Patienten, d. h. Bestimmung des statischen Patientendrucks,
   • Gravimetrische Entleerung des Dialysats aus dem Patienten in einen Messbeutel,
   • Höheneinmessung des Beutels dadurch, dass nach einer bestimmten Zeit der Dialysatvolumenstrom gestoppt wird und der Beutel ebenfalls statisch eingemessen wird,
   • Volumenbilanzierung des Inhalts des Messbeutels bei Entleerung des Messbeutels und
   • Berechnung des Katheterflußwiderstandes aus den ermittelten Parametern Volumen und Zeit.

8. Vorrichtung nach Anspruch 7, **dadurch gekennzeichnet, dass** zur Bestimmung des statischen Druckes des Patienten ein erstes Ventil (16) geöffnet wird, während ein zweites Ventil (18) sowie ein drittes Ventil (20) geschlossen bleibt.

9. Vorrichtung nach Anspruch 7 oder 8, **dadurch gekennzeichnet, dass** das Dialysat vom Patienten für eine bestimmte Zeit (x) gravimetrisch entweder über das erste Ventil (16), die Pumpenkammer der in der Leitung ebenfalls angeordneten Pumpe (22) sowie das zweite Ventil (18) oder über eine Bypassleitung (24) und das in dieser angeordnete dritte Ventil (20) erfolgt.

10. Vorrichtung nach Anspruch 9, **dadurch gekennzeichnet, dass** die Zeitspanne (x) zur gravimetrischen Entleerung so gewählt ist, dass eine Komplettentleerung vermieden wird.

11. Vorrichtung nach einem der Ansprüche 7 bis 10, **dadurch gekennzeichnet, dass** zur Ermittlung des statischen Drucks das zweite Ventil (18) geöffnet wird, während das erste Ventil (16) und das dritte Ventil (20) geschlossen sind, und dass der statische Druck des Beutels über einen Drucksensor (24) gemessen wird.

12. Vorrichtung nach einem der Ansprüche 7 bis 10, **dadurch gekennzeichnet, dass** das Dialysatvolumen dadurch ermittelt wird, dass der Messbeutel mit Hilfe der Pumpe (22) in eine Drainage (d) entleert wird und über eine Bilanzierungseinheit bilanziert bzw. gemessen wird.

## Claims

1. A device for peritoneal dialysis with a means for the regular discharge and reuptake of dialysate, which comprises a conduit with a catheter,
wherein a measurement bag is present, in which dialysate can be received, wherein first means are provided, with which the static pressure in the conduit between catheter and measurement bag can be determined, wherein second means are provided, by means of which the dialysate volume flow can be introduced intro the measurement bag for a certain period, wherein third means are present, with which the volume of the dialysate introduced into the measurement bag can be determined; **characterized in that** fourth means are present, by which the catheter resistance can be calculated from the values determined by the first to third means.

2. The device according to claim 1, **characterized in that** the first means comprise a pressure sensor.

3. The device according to claim 1 or 2, **characterized in that** the second means include at least one time-controllable valve.

4. The device according to any of claims 1 to 3, **characterized in that** the third means include a pump and a balancing unit for determining the dialysate volume.

5. The device according to any of claims 1 to 4, **characterized in that** in the conduit between catheter and measurement bag a first valve, a pressure sensor, a pump and a second valve are disposed.

6. The device according to claim 5, **characterized in that** a bypass conduit in which a third valve is disposed is branched off from the conduit before the first valve, and that the bypass conduit again opens into the conduit after the second valve.

7. The device according to any of the preceding claims, wherein by means of the device the catheter resistance can automatically be determined with the following steps:

   • levelling of the patient, i.e. determination of the static patient pressure;
   • gravimetric drainage of the dialysate from the patient into a measurement bag,
   • levelling of the bag by stopping the dialysate volume flow after a certain period and likewise statically levelling the bag;
   • volume balancing of the contents of the measurement bag when draining the measurement bag; and
   • calculation of the catheter flow resistance from the determined parameters volume and time.

8. The device according to claim 7, **characterized in that** for determining the static pressure of the patient a first valve (16) is opened, while a second valve (18) and a third valve (20) remain closed.

9. The device according to claim 7 or 8, **characterized in that** the dialysate is gravimetrically drained from the patient for a certain period (x) either via the first valve (16), the pump chamber of the pump (22) likewise disposed in the conduit and the second valve (18), or via a bypass conduit (24) and the third valve (20) disposed in the same.

10. The device according to claim 9, **characterized in that** the period (x) for gravimetric drainage is chosen such that a complete drainage is avoided.

11. The device according to any of claims 7 to 10, **characterized in that** for determining the static pressure the second valve (18) is opened, while the first valve (16) and the third valve (20) are closed, and that the static pressure of the bag is measured via a pressure sensor (24).

12. The device according to any of claims 7 to 10, **characterized in that** the dialysate volume is determined **in that** the measurement bag is drained into a drainage (d) by means of the pump (22) and balanced and measured via a balancing unit.

**Revendications**

1. Dispositif pour une dialyse péritonéale avec une installation pour la distribution et récupération régulière de dialysat, qui comprend un conduit avec un cathéter, où un sac de mesure est prévu dans lequel le dialysat peut être reçu, en ce que des premiers moyens sont prévus par lesquels la pression statique dans le conduit entre le cathéter et le sac de mesure peut être déterminée, en ce que des deuxièmes moyens sont prévus au moyen desquels le flux volumique du dialysat peut être guidé pendant une durée déterminée dans le sac de mesure, en ce que des troisièmes moyens sont prévus par lesquels le volume du dialysat amené dans le sac de mesure peut être déterminé; **caractérisé en ce que** des quatrième moyens sont prévus par lesquels la résistance du cathéter peut être calculée à partir des valeurs déterminées par les premiers à troisièmes moyens.

2. Dispositif selon la revendication 1, **caractérisé en ce que** les premiers moyens comprennent un capteur de pression.

3. Dispositif selon la revendication 1 ou 2, **caractérisé en ce que** les deuxièmes moyens présentent au moins une vanne apte à être commandée dans le temps.

4. Dispositif selon l'une des revendications 1 à 3, **caractérisé en ce que** les troisièmes moyens présentent une pompe et une unité d'établissement de bilan pour déterminer le volume du dialysat.

5. Dispositif selon l'une des revendications 1 à 4, **caractérisé en ce que** sont disposés dans le conduit entre le cathéter et le sac de mesure une première vanne, un capteur de pression, une pompe et une deuxième vanne.

6. Dispositif selon la revendication 5, **caractérisé en ce que** part du conduit en amont de la première vanne un conduit de dérivation dans lequel est disposé une troisième vanne, et **en ce que** le conduit de dérivation, en aval de la deuxième vanne, débouche à nouveau dans le conduit.

7. Dispositif selon l'une des revendications précédentes, où au moyen du dispositif, la résistance du cathéter peut être déterminée automatiquement par les étapes suivantes:

   • mesure de hauteur du patient, c'est-à-dire détermination de la pression statique du patient,
   • vidage gravimétrique du dialysat du patient dans un sac de mesure,
   • mesure de hauteur du sachet en ce que, après un temps déterminé, le flux volumique du dialysat est arrêté et le sachet est également mesuré de manière statique,
   • équilibrage de volume du contenu du sac de mesure lors du vidage du sac de mesure et
   • calcul de la résistance d'écoulement du cathéter à partir des paramètres déterminés volume et temps.

8. Dispositif selon la revendication 7, **caractérisé en ce que** pour la détermination de la pression statique du patient, une première vanne (16) est ouverte tandis qu'une deuxième vanne (18) ainsi qu'une troisième vanne (20) restent fermées.

9. Dispositif selon la revendication 7 ou 8, **caractérisé en ce que** le dialysat du patient a lieu pour une durée déterminée (x) de manière gravimétrique soit par la première vanne (16), la chambre de pompe de la pompe (22) également disposée dans le conduit ainsi que la deuxième vanne (18) ou par un conduit de dérivation (24) et la troisième vanne (20) disposée dans celui-ci.

10. Dispositif selon la revendication 9, **caractérisé en ce que** la durée (x) pour le vidage gravimétrique est sélectionnée de façon à éviter un vidage complet.

11. Dispositif selon l'une des revendications 7 à 10, **caractérisé en ce que** pour la détermination de la pression statique, la deuxième vanne (18) est ouverte pendant que la première vanne (16) et la troisième vanne (20) sont fermées, et **en ce que** la pression statique du sac est mesurée par un capteur de pression (24).

12. Dispositif selon l'une des revendications 7 à 10, **caractérisé en ce que** le volume de dialysat est mesuré **en ce que** le sac de mesure est vidé à l'aide de la pompe (22) dans un drainage (d) et, par une installation d'équilibrage, est équilibré respectivement mesuré.

# Fig.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5141493 A